# EUROPEAN PATENT APPLICATION

(11) **EP 4 740 896 A1**
(43) Date of publication of application: **13.05.2026**
(21) Application number: 24211946.9
(22) Date of filing: 10.11.2024
(51) Int. Cl.: A61B 90/70, A61M 25/00

(54) **MEDICAL CATHETER CLEANING WIRE CLAMPING APPARATUS AND MEDICAL CATHETER CLEANING APPARATUS**

(71) Applicant: CaDo Medial Solutions GmbH, 91052 Erlangen (DE)
(72) Inventor: Elsässer, David, 91058 Erlangen (DE); Wiehl, Michael, 91052 Erlangen (DE); Wenzel, Erich Michael, 91341 Röttenbach (DE)
(74) Representative: Wittmann, Günther

(57) **Abstract**

The present invention discloses a medical catheter cleaning wire clamping apparatus, comprising
- a clamping device adapted to clamp the cleaning wire;
- a cleaning wire detection device adapted to detect that the cleaning wire is introduced into the clamping device;
- a translational drive configured to drive the clamping device in the horizontal direction;
- a rotational drive configured to rotate the clamping device around its rotational axis;
- a securing device configured to engage with the securing device; and
- a controller configured to
- instruct the translational drive to move the clamping device towards the securing device until the clamping device engages with the securing device; and to
- instruct the rotational drive to rotate the clamping device around its longitudinal direction until the cleaning wire is clamped, after the cleaning wire detection device detected insertion of the cleaning wire.

The invention also discloses a medical catheter cleaning apparatus, comprising
- a housing having an insertion opening for inserting a cleaning wire; and
- the medical catheter cleaning apparatus positioned in the housing.

## Description

The present invention relates to a medical catheter cleaning wire clamping apparatus and a medical catheter cleaning apparatus, particularly for cleaning urine catheters.

### Field of the Invention

Catheters for draining the bladder of a human are known since ancient times. Catheters are used, if a patient cannot drain his bladder. Catheters can be inserted transurethral or suprapubic.

A frequent problem in use of catheters is that the lumen of the catheter may get obstructed, such as by salt. Generally, such obstructions occur in the proximal portion of the catheter close to the inlet opening of the catheter.

If a permanent catheter gets obstructed, the patient will feel strong pain requiring acute intervention. In the prior art the catheter may be flushed back, e.g. by a syringe. In many cases the obstruction cannot be eliminated by flushing. Consequently, the catheter has to be changed under emergency conditions by a physician.

If the catheter is not changed as early as possible under emergency conditions, additional complications may develop by the patient, such as sepsis, kidney failure, inflammation of the urethra or the like.

Currently no catheter cleaning apparatus for the cleaning of an catheter connected to a patient is commercially available.

### Prior art

WO 87/02255 discloses an ultrasonic self-cleaning catheter system.

US 5,030,213 discloses a catheter router assembly for cleaning salt blockages in a previously inserted catheter. A length of flexible, stainless steel cable is provided and a silver solder tip is applied at the proximal end of the cable. The silver solder tip is then machined to form a suitable cutting edge for cutting or drilling through the body salts which clog the catheter.

US 2005/0267421 A1 discloses a catheter cleaner comprising a head, a rod and a handle. The head defines a tip and cutting edges. The tip may have a curved configuration which may be operative to assist the insertion of the cleaner into an indwelling catheter.

US 6,183,450 B1 discloses a catheter cleaning device for cleaning a catheter of obstructions. The catheter cleaning device comprises an elongate hollow housing, a guide member encircling the housing and including a bar extending across a diameter thereof and through first and second slots of the elongate hollow housing. A guide wire extends from the bar.

### Summary of the invention

It is an object of the present invention to provide a medical catheter cleaning wire clamping apparatus that can be operated safely by an operator that has passed only minimal training.

The object of the present invention is solved by a medical catheter cleaning wire clamping apparatus according to claim 1. The present disclosure discloses a medical catheter cleaning wire clamping apparatus comprising a clamping device adapted to clamp the cleaning wire and a cleaning wire detection device adapted to detect that the cleaning wire is introduced into the clamping device. The medical catheter cleaning wire clamping apparatus further comprises a rotational drive configured to rotate the clamping device around its longitudinal axis. The medical catheter cleaning wire clamping apparatus further comprises a securing device configured to engage with the securing device.

The medical catheter cleaning wire clamping apparatus further comprises a controller configured to instruct the translational drive to move the clamping device towards the securing device until the clamping device engages with securing device. The controller is further configured to instruct the rotational drive to rotate the clamping device around its longitudinal direction until the cleaning wire is clamped, after the cleaning wire detection device detected insertion of the cleaning wire.

It is an advantage of the present invention that the cleaning wire is automatically clamped, when the operator inserts the distal end of the cleaning wire into the opening of the medical catheter cleaning wire clamping apparatus and into the clamping device. This ensures that the cleaning wire can be safely clamped by the medical catheter cleaning wire clamping apparatus after minimal training of the operator.

The clamping device of the catheter clamping apparatus may comprise a chuck having a plurality of jaws and a clamping nut positioned around the jaws. If the clamping nut is rotated with the respect to the jaws or verse vice, the jaws can grip an object or release an object, since the distance of the jaws to each other is reduced by rotating the clamping nut in one direction and the distance between the jaws is increased by rotating the clamping nut in the opposite direction.

The securing device may be adapted to inhibit rotation of the clamping nut if the clamping nut engages with the securing device.

In one embodiment the clamping nut may comprise a recess adapted to engage in a protrusion of the securing device.

In another embodiment the clamping nut may comprise a protrusion adapted to engage in a recess of the securing device.

The securing device of the medical catheter cleaning wire clamping apparatus may comprise a securing device opening through which the cleaning wire passes when it is introduced into the housing of the clamping device.

In one embodiment the securing device opening of the securing device may comprise at least partially and essentially conical shape to facilitate insertion of the cleaning wire. The securing device may have an inward tapered shape.

The cleaning wire detection device may comprise a cleaning wire stop configured to stop the cleaning wire inserted into the clamping device. The cleaning wire detection device may further comprise an elastic element mechanically coupled with the cleaning wire stop. The cleaning wire detection device may further comprise a detector having an open state and a closed state. If the cleaning wire is pressed against the cleaning wire stop, the cleaning wire stop is pressed towards the elastic element and the detector switches from its first state to its second state. Thereby, a reliable detection of the insertion of the wire into the clamping device can be detected. It is to be understood that any mechanically component may be arranged between the cleaning wire stop and the detector. The second state of the detector indicates that the cleaning wire detection device has detected the insertion of the cleaning wire into the clamps.

In one embodiment, the detector or may be an electric switch.

In another embodiment the cleaning wire detection device or the detector or may be a light barrier.

The present disclosure also discloses a medical catheter cleaning apparatus, comprising a housing having an insertion opening for inserting a cleaning wire and the medical catheter cleaning wire clamping apparatus described above and positioned in the housing. The translational drive is positioned in the housing of the medical catheter cleaning apparatus.

During introducing the wire, the wire passes through the insertion opening in the housing and the securing device opening in the stop of the medical catheter cleaning wire clamping apparatus.

The controller may be configured to instruct the translational drive to move the cleaning wire in the translational direction to clean the catheter, if the cleaning wire is clamped by the clamping device. Additionally or alternatively, the controller may be adapted to instruct the rotational drive to rotate the clamps around their longitudinal axis. Thereby, the inner of the catheter is cleaned by the cleaning head of the cleaning wire. It is to be understood that the cleaning head my have protrusion with a shape suitable for removing obstructions in the catheter.

The controller may be further configured to determine an identification of the cleaning wire. The controller may determine based on the identification of the cleaning wire, whether the cleaning wire has already been used. If the controller determines that the cleaning wire has already been used, the operation is interrupted until an identification of another cleaning wire is determined. If the controller determines that the cleaning wire has not been used, the controller waits until the cleaning wire detection device detects insertion of the cleaning wire. The controller is configured to mark the identification of the cleaning wire as used in a database, if cleaning of the catheter has been finished. For hygienic reasons, a cleaning wire must only be used once and must not be used a further time, particularly not for a different patient.

The medical catheter cleaning apparatus may further comprise a configuration sensor coupled with the controller. The controller may be configured to instruct the configuration sensor to read a configuration identifier, if the configuration sensor senses the presence of a configuration identifier media. The controller may be configured to request the operator to enter the catheter type that shall be cleaned. The controller may be configured to store the catheter type that shall be cleaned as a set catheter type in a non-volatile memory. The medical catheter cleaning wire clamping apparatus is configured that it can only claim one type of catheter.

Since the translational drive of the catheter cleaning apparatus moves the cleaning wire a predetermined and mechanically limited length and since the blockage is at the end of the catheter in the body of the patient and it must be insured to clean exactly the obstructed portion in the catheter. Therefore, the operation of the controller and the cleaning wire must match the catheter. Otherwise, a cleaning head of the cleaning wire may either not go in far enough or overshoot the target and potentially injure the patient.

The catheter cleaning apparatus may be configured by the controller and the configuration identifier media when it is used for the first time. Therefore, the catheter length and diameter must be transmitted. Before cleaning a catheter, the user scans a identification of a catheter. This allows the controller to compare whether the cleaning wire matches the catheter to be cleaned. This is an important safety feature and is not absolutely necessary for operation. Thereby, injury of the patient can be avoided.

The configuration sensor may be an NFC sensor and/or a RFID sensor. The configuration identifier media may be a RFID card and/or a communication device comprising an NFC transmitter.

The controller may be configured to request the operator to enter the actual catheter type. If the actual catheter type corresponds to the set catheter type the identification of the cleaning wire is determined by the controller. The controller is configured to, if the actual conceded type corresponds not to the set catheter type or if the cleaning wire is marked as used in the database, wait, until the identification of another cleaning wire is determined. Thereby, the controller ensures that only the correct catheter type is cleaned and that each cleaning wire is only used once.

The controller may be configured to request the configuration sensor to sense presence of the configuration identifier media. If presence of the configuration identifier media is sensed by the configuration sensor, the controller may instruct the configuration sensor to read the configuration identifier from the configuration identifier media. Thereafter, the controller verifies, whether the set catheter type is stored in the non-volatile memory. If the set catheter type is stored in the non-volatile memory, the operator is requested to enter the actual catheter type. If no catheter type is stored in the non-volatile memory, the controller requests the configuration sensor to sense the configuration identifier media. If the configuration sensor senses the configuration senso media, The operator is requested to enter the type of catheter that shall be cleaned. Thereby the controller ensures that only the appropriate type of catheter can be cleaned by the cleaning wire.

### Brief Description of the Drawings

The invention is now described more detailed reference to the drawings, showing exemplary and non-limiting embodiments, wherein
Figure 1 illustrates a cut away view of the medical catheter cleaning apparatus during introduction of the cleaning wire;
Figure 2 illustrates a cut away view of the inventive medical catheter cleaning apparatus before the cleaning wire is locked by the clamps of the chuck; and
Figure 3 illustrates a cut away view of the medical catheter cleaning apparatus according to the present invention, after the wire has been clamped by the clamps of the chuck.

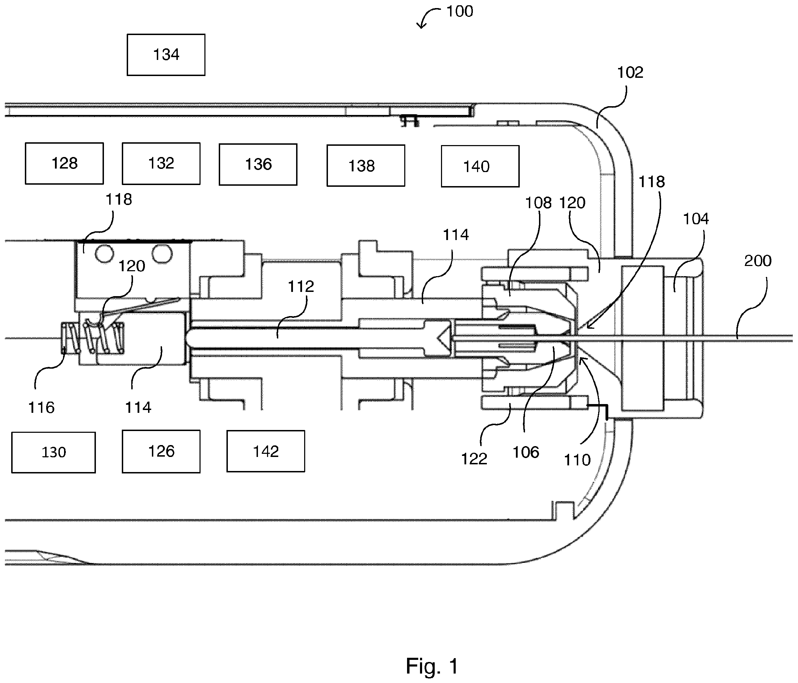

Reference is made to figure 1 showing a cut view of the medical catheter cleaning apparatus 100 in the open state. The medical catheter cleaning apparatus 100 comprises a housing 102 with an insertion opening 104 through which a distal end of a catheter cleaning wire 200 is inserted. The distal end of the catheter cleaning wire 200 passes through a securing device opening 118 of a securing device 120 to be inserted between clamps 106 of a chuck 106, 108. The chuck 106, 108 may comprise at least three clamps 106. A clamping nut 106 is positioned around the clamps 108.

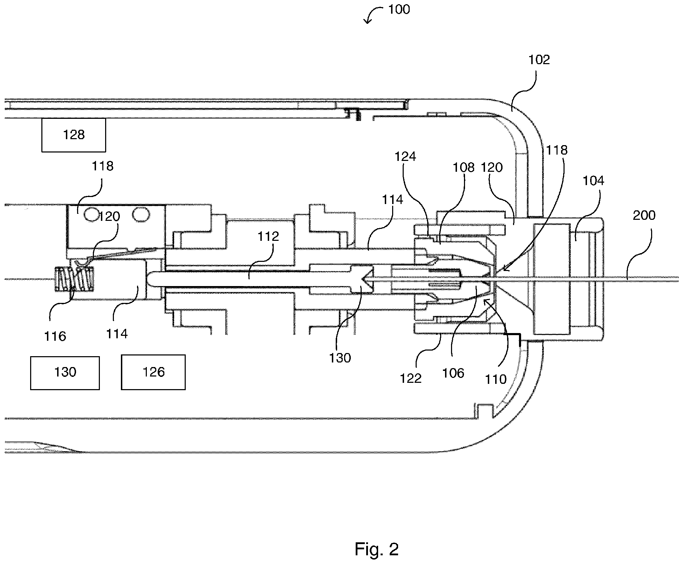

Reference is now made to figure 2 showing a cut away view of the medical catheter cleaning apparatus 100 at the beginning of the process of closing the clamps 106. The distal end of the catheter cleaning wire 200 has been pushed into the housing 102 of the medical catheter cleaning apparatus 100 until it contacts a wire stop 130. The wire stop 130 is arranged in a guide 114 such that the wire stop 130 can only be moved in one translational direction. The wire stop 130 is mechanically coupled with an extension 112 that contacts mechanically a slidable trigger element 114. The trigger element 114 is biased translationally against the extension 112 and the wire stop 130 by an elastic element 116 such as a spring. If the cleaning wire 200 is moved further inwards, the wire stop 130, the extension 112 and the trigger element 114 is pressed against the spring 116, such that the spring 116 is compressed. Thereby, the trigger element 114 actuates an actuation element 120 of a switch 118. Thereby, the switch 118 switches from its first state to its second state. The first state of the switch 118 may be the open state, and the second state of the switch 118 may be the closed state. The second state of the switch 118 indicates that the cleaning wire 200 is positioned between the clamps 106 of the chuck 106, 108.

During the process of closing the clamps 106 the chuck 106, 108 is positioned in the securing device 120, which as a generally cylindrical opening. The securing device 120 comprises a protrusion 124, such as a nib or gill that protrudes towards the clamping nut 108 of the chuck 106, 108. In the embodiment shown in figure 2 the protrusion 124 protrudes radially inward. The clamping nut 108 comprises a recess, such as a groove.

During the process of closing the clamps 106 the clamping nut 108 is positioned such in the securing device 120 that the protrusion 124 of the securing device 120 is positioned in the recess in the clamping nut 108. Then, a controller 128 instructs a rotational drive 126 to rotate the clamps 106 in a first rotational direction. Thereby, the clamping nut 108 is moved towards the center of the medical catheter cleaning apparatus 100 in other words, an inward tapered potion 110 of the clamping nut 108 is moved on conical portions of the clamps 106 and compresses the clamps 106, such that the clamps 106 grip the cleaning wire 200.

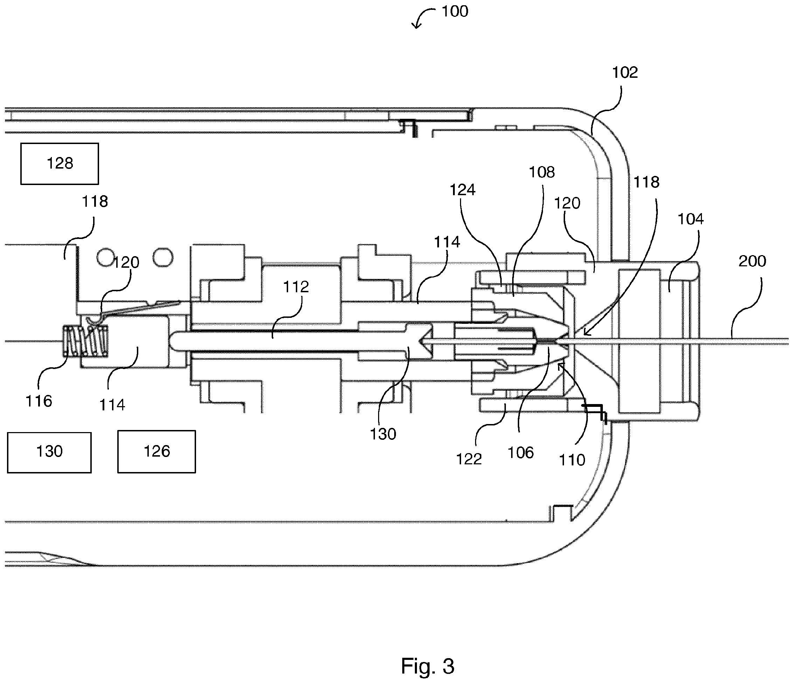

Reference is made to figure 3, showing the catheter cleaning drive apparatus in the state, in which the clamps 106 clamp the cleaning wire 200. The clamping nut 108 presses with its inward tapered portion 110 the conical portions of the clamps 106 against the cleaning wire 200.

Thereafter, the controller 128 instructs a translational drive one 128 to move the clamps away from the insertion opening 104. Thereafter, the operator may insert the cleaning wire 200 into the catheter that is connected with a patient. It is to be understood that the cleaning wire 200 may comprise cleaning elements and its proximal end on a cleaning head.

Thereafter, the controller 128 instructs the translational drive 130 to move the clamps 106 towards the opening and instructs the rotational drive 126 to rotate the clamps 106 around its rotational axis. It is to be understood, that the controller 128 must instruct the translational drive 130 to stop movement of the clamps 106 before the recess in the clamping nut 108 reaches the protrusion 124 of the securing device 120 When cleaning of the interior of the catheter is finished, the controller 128 instructs the translational drive 1 30 to position the chuck 106, 108 as shown in figure 3, i. e. the recess of the clamping nut 108 engages with the protrusion 124 of the securing device 120. Then, the controller 128 instructs the rotational drive 126 to rotate the clamps 106 and a second rotational direction opposite to the first rotational direction. Then, the clamping nut 108 is released from the clamps 106. This state is shown in figure 2. Now the operator can remove the cleaning wire 200 from the clamps 106.

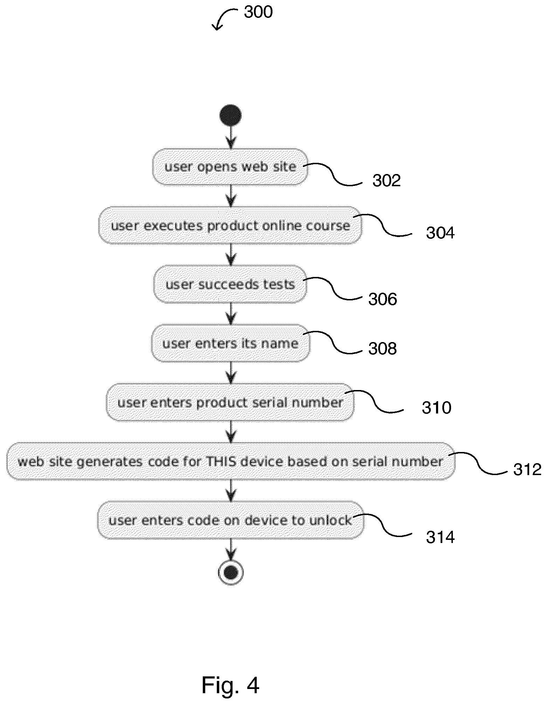

Reference is made to figure 4 showing a method of training an operator of the medical catheter cleaning apparatus 100 according to the present invention. The operator has to open a web site in step 302, and has to attend an online course in step 304. Thereafter, the operator has to succeed a test in step 306. In step 308 the operator enters its name, and in step 310 the operator enters the serial number of the medical catheter cleaning apparatus 100. The method generates a code for the particular medical catheter cleaning apparatus based on the serial number in step 312. In step 314, the operator enters the code by input means 136, such as input buttons in the particular medical catheter cleaning apparatus 100 to unlock it.

This ensures that only operators who have been trained in operating the medical catheter cleaning apparatus 100 operate it.

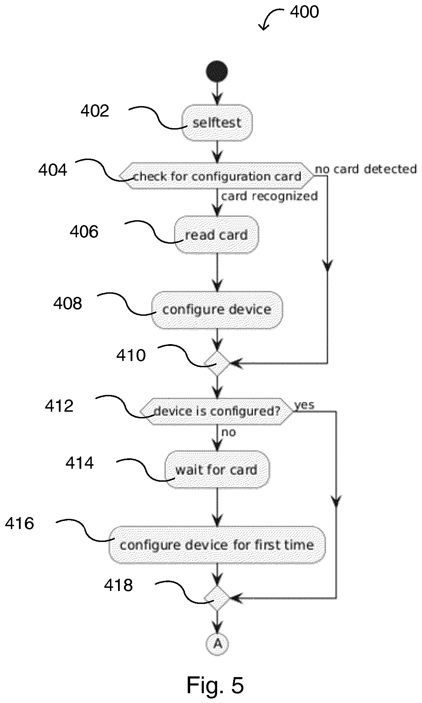

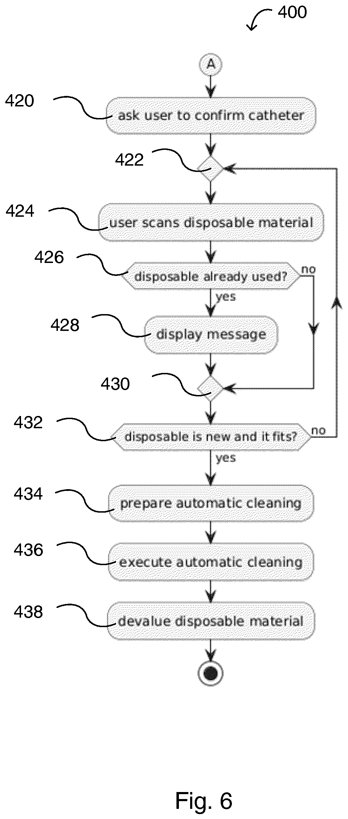

Reference is made to figure 5 and 6 showing the method 400 of operating the medical catheter cleaning apparatus 100 according to the present invention.

In step 402 the controller 128 medical performs a self-test of the medical catheter cleaning apparatus 100. In step 404 the controller instructs the configuration sensor 132, whether an RFID card, 134 (configuration identifier media) can be detected. If the configuration sensor 132 detects the RFID card 134 (see figure 1), the controller 128 instructs the configuration sensor to read the configuration identifier from the configuration identifier media. The operator may input in step 406 a new configuration identifier by the input means 136, such as input buttons. The configuration identifier in the context of this disclosure is the catheter type. Currently, four types of catheters are available. Each catheter type requires a different catheter cleaning wire 200.

In step 408, the operator can configure the medical catheter cleaning apparatus 100 for a new catheter type, if the medical catheter cleaning apparatus 100 was already configured. The new catheter type is stored in a non-volatile memory 142.

If in step 404 no RFID card was detected, the method proceeds via step 410 to step 412 and the controller checks, whether the medical catheter cleaning apparatus 100 is already configured. If the medical catheter cleaning apparatus 100 is not configured as determined by the controller 128, the controller 128 instructs the configuration sensor 132 in step 414 to wait until it senses the RFID card. If the configuration sensor 132 senses the RFID card 134, the controller displays in step 416 a configuration menu on a display and the operator can enter the catheter type by the input means 136. The entered catheter type is stored in the non-volatile memory 142. Then, the method proceeds via step 418 to step 420.

If the controller 128 determines in step 412 that the medical catheter cleaning apparatus 100 is already configured, the method while step 418 to step 420.

In step 420 the controller 128 asks the operator, which catheter type shall be cleaned. The operator must enter the catheter type via the input buttons 136. The method proceeds via step 422 to step 424, in which the operator must scan a part of the cleaning wire 200 or its packing by the imaging device 138. The imaging device 138 may be a camera, a barcode scanner or any other suitable imaging device. The part of the cleaning wire 200 or its packaging that is scanned may be a barcode, a QR code or the like.

In step 426 the controller 128 determines, whether the cleaning wire 200 has already been used for cleaning a catheter. If the controller determines that the cleaning wire 200 has already been used, the controller 128 displays a message on the display 140 indicating that the cleaning wire 200 has already been used and must not be reused.

Then, the method proceeds via step 430 to step 432.

If the controller determines in step 426 that the cleaning wire 200 has not been used, the method proceeds via step 430 to step 432. If the controller determines in step 432 that the cleaning wire has already been used or is from a not configured catheter type as compared with the set catheter type stored in the non-volatile memory 142, the method returns via step 422 to step 424, in which the controller requests the operator to scan a part of the cleaning wire or its packaging. The method continues as described above.

If the processor determines in step 432 that the cleaning wire 200 has not been used and is of the configured catheter type, the method proceeds to step 434, in which the automatic cleaning is prepared. The step of preparing automatic cleaning of the catheter may include the above described the steps of clamping the cleaning wire 200. This step may include that the controller 128 verifies, whether the chuck 106, 108 is positioned in the securing device 120, 124 and that the protrusion 124 engages in the recess in the clamping nut 108.

In step 436, the controller controls the automatic cleaning by instructing the translational drive 130 to move the clamps 106 translationally and by optionally instructing the rotational drive 126 to rotate the clamps 106 and thereby rotate the cleaning wire 200. When cleaning of the catheter is finished, the controller instructs the rotational drive 126 to position the clamping nut 108 such that the protrusion 124 of the securing device 120 can engage with the recess in the clamping nut 108 and instructs the translational drive 130 to position the chuck 106, 108 in the securing device 120. Then, the clamps 106 are opened as described above and the operator can remove the cleaning wire 200 from the catheter cleaning apparatus 100.

It is an advantage of the present invention that an operator, such as nursing staff can clean a catheter with the medical catheter cleaning apparatus 100 according to the invention after a short training. Generally, no physician is required for operating the medical catheter cleaning apparatus 100 according to the present invention. Further, considerably time is saved by cleaning a catheter as compared to changing a catheter.

## Claims

1. A medical catheter cleaning wire clamping apparatus, comprising
- a clamping device adapted to clamp the cleaning wire;
- a cleaning wire detection device adapted to detect that the cleaning wire is introduced into the clamping device;
- a translational drive configured to drive the clamping device in the horizontal direction;
- a rotational drive configured to rotate the clamping device around its rotational axis;
- a securing device configured to engage with the securing device; and
- a controller configured to
- instruct the translational drive to move the clamping device towards the securing device until the clamping device engages with the securing device; and to
- instruct the rotational drive to rotate the clamping device around its longitudinal direction until the cleaning wire is clamped, after the cleaning wire detection device detected insertion of the cleaning wire.

2. The medical catheter cleaning wire clamping apparatus according to claim 1, wherein
- the clamping device is a chuck comprising a plurality of jaws and a clamping nut positioned around the plurality of jaws.

3. The medical catheter cleaning wire clamping apparatus according to claim 2, wherein the securing device is adapted to inhibit rotation of the clamping nut if the clamping nut engages with the securing device.

4. The medical catheter cleaning wire clamping apparatus according to claim 3, wherein
- the clamping nut comprises a recess adapted to engage in a protrusion of the securing device; and/or
- the clamping nut comprises a protrusion adapted to engage in a recess of the securing device.

5. The medical catheter cleaning wire clamping apparatus according to any one of claims 1 to 4, wherein the securing device comprises
- a securing device opening through which the cleaning wire passes when it is introduced into the clamping device.

6. The medical catheter cleaning wire clamping apparatus according to any one of claims 1 to 5, wherein the cleaning wire detection device comprises
- a cleaning wire stop configured to stop the cleaning wire inserted in the clamping device;
- an elastic element mechanically coupled with the cleaning wire stop;
- a detector having a first state and a second state, wherein, if the cleaning wire is pressed against the cleaning wire stop, the cleaning wire stop is pressed against the elastic element and the detector switches from its first state to its second state,
- wherein the second state indicates the insertion of the cleaning wire.

7. The medical catheter cleaning wire clamping apparatus according to claim 6, wherein the detector is an electric switch.

8. The medical catheter cleaning wire clamping apparatus according to claim 6, wherein the detector is a light barrier.

9. A medical catheter cleaning apparatus, comprising
- a housing having an insertion opening for inserting a cleaning wire; and
- the medical catheter cleaning apparatus according to any one of claims 1 to 8 positioned in the housing.

10. The medical catheter cleaning apparatus according to claim 9, wherein the controller is configured
- to instruct the translational drive to move the cleaning wire longitudinal direction to clean the catheter, if the cleaning wire is clamped by the clamping device; and/or
- instruct the rotational drive to rotate the clamps around their longitudinal axis.

11. The medical catheter cleaning apparatus according to claims 8 or 9, wherein the controller is configured to:
- determine an identification of the cleaning wire;
- determine based on the identification, whether the cleaning wire has already been used;
- if it is determined that the cleaning wire has already been used, the operation is interrupted until an identification of a new cleaning wire is determined;
- if it is determined that the cleaning wire has not been used, to wait until the cleaning wire detection device detects insertion of the cleaning wire; and
- marking the cleaning wire as used in a data base, if cleaning of the catheter has been finished.

12. The medical catheter cleaning apparatus according to claim 11, further comprising a configuration sensor;
wherein the controller is configured to
- to instruct the configuration sensor to read a configuration identifier from a configuration identifier media, if the configuration sensor senses the presence of the configuration identifier media;
- to request an operator to enter the catheter type that shall be cleaned;
- to store the catheter type that shall be cleaned as a set catheter type in a non-volatile memory of the medical catheter cleaning apparatus.

13. The medical catheter cleaning apparatus according to claim 12, wherein the configuration sensor is at least one of:
- an NFC sensor;
- a RFID sensor;
wherein the configuration identifier media is at least one of:
- a RFID card;
- a communication device comprising an NFC transmitter.

14. The medical catheter cleaning apparatus according to claim 12 or 13, wherein the controller is configured to:
- request the operator to enter the actual catheter type;
- if the actual catheter type corresponds to the set catheter type, the identification of the cleaning wire determined; and
- if the actual catheter type corresponds not to the set catheter type or if the cleaning wire is marked as used in the data base, the identification of another cleaning wire is determined.

15. The medical catheter cleaning apparatus according to any one of claims 11 to 14, wherein the controller is configured to
- request the configuration sensor to sense the configuration identifier media;
- if the configuration identifier media is sensed by the configuration sensor, to instruct the configuration sensor to read the configuration identifier from the configuration identifier media;
- if the configuration identifier media is sensed by the configuration sensor, to verify, whether the set catheter type is stored in the non-volatile memory;
- if the set catheter type is stored in the non-volatile memory, the operator is requested to enter the actual catheter type; and
- if the set catheter type is not stored in the non-volatile memory, request the configuration sensor to sense the configuration identifier media.
